# EUROPEAN PATENT APPLICATION

(11) **EP 4 667 010 A1**
(43) Date of publication of application: **24.12.2025**
(21) Application number: 25181981.9
(22) Date of filing: 11.06.2025
(51) Int. Cl.: A61K 35/28, A61P 13/10, C12N 5/0775

(54) **CELL COMPOSITION FOR USE IN THE TREATMENT OF DETRUSOR UNDERACTIVITY AND METHOD FOR PREPARING THE SAME**

(30) Priority: 17.06.2024 US 202463660589 P
(71) Applicant: MetaTech (AP) Inc., New Taipei City 221 (TW)
(72) Inventor: CHUEH, Shih-Chieh, 211 New Taipei City (TW); CHANG, Shang-Jen, 211 New Taipei City (TW); CHEN, Jing-Yun, 211 New Taipei City (TW); YANG, Ying-Chen, 211 New Taipei City (TW); CHEN, Tsung-Chi, 211 New Taipei City (TW); CHEN, Kuan-Jung, 211 New Taipei City (TW)
(74) Representative: Wang, Bo

(57) **Abstract**

The present invention provides a cell composition for use in the treatment of detrusor underactivity and a method for preparing the same. The cell composition comprises a cell spheroid formed by seeding a plurality of mesenchymal stem cells in a culture device comprising a plurality of culture wells, wherein each of the culture wells has a surface coated with a low-protein-adhesion layer, and culturing the cells under the conditions to induce spheroid formation. Through the cell composition of the present invention, three-dimensional structured cell spheroids are formed to promote intercellular interactions and physiological function expression, thereby enhancing cell survival rates and functional performance after transplantation. In particular, the cell composition effectively facilitates the repair and restoration of contractile function of bladder muscle tissue, thereby improving the clinical symptoms associated with detrusor underactivity.

## Description

### BACKGROUND OF THE INVENTION

### 1. Field of the invention

The present invention relates to a cell composition, and more particularly, to a cell composition for use in the treatment of detrusor underactivity, as well as a method for preparing the cell composition.

### 2. Description of the prior art

Detrusor underactivity (DU) is a common and complex urological dysfunction. Its etiology may be associated with factors such as neurological injury, muscle degeneration, postoperative complications, or metabolic disorders. Clinically, DU is characterized by difficulties in urination, weak urinary stream, or dependence on catheterization, all of which significantly impair the patient's quality of life. With the trend of an aging population, the incidence of detrusor underactivity continues to rise, making it an urgent clinical issue in the field of urology.

Current clinical treatment options mainly include pharmacological therapy, physical stimulation training, and invasive surgical interventions, each with significant drawbacks and limitations:
1. Pharmacological therapy involves the use of agents such as acetylcholine enhancers and parasympathomimetic drugs to stimulate bladder contraction. However, these often provide only short-term symptom relief and may cause side effects such as nausea, diarrhea, or arrhythmia. Moreover, these drugs cannot repair degenerated or damaged muscle and nerve tissues, limiting their efficacy in moderate to severe cases.
2. Physical therapy includes bladder training, pelvic floor exercises, and electrical stimulation therapy. These approaches require long-term execution and typically show slow therapeutic progress. Furthermore, they do not directly restore bladder muscle function and are often ineffective in patients with severe atrophy or neurological deficits.
3. Invasive treatments, such as long-term catheterization, bladder augmentation surgery, or implantation of neuromodulation devices, may temporarily improve voiding function. However, they are associated with risks of infection, complications, patient dependency, and require specialized procedures with high costs, thus affecting long-term patient compliance.

With the advancement of regenerative medicine and stem cell therapy technologies, cell therapies are emerging as a promising strategy for the treatment of detrusor underactivity. Conventional two-dimensional (2D) cell culture remains the most widely used method for preparing cells, yet it fails to mimic the in vivo three-dimensional (3D) microenvironment. This limitation restricts intercellular signaling and tissue architecture formation, resulting in suboptimal cellular function, reduced post-transplantation survival, and diminished therapeutic efficacy. Furthermore, cells are vulnerable to mechanical damage during handling and transplantation, which further compromises their performance. These limitations are especially critical in the context of treating detrusor underactivity, where current technologies fail to effectively promote regeneration of bladder smooth muscle tissue and restoration of normal voiding function.

Therefore, there is an urgent need for a new type of cell therapy agent and its preparation method, which can more realistically mimic the three-dimensional structure of the body, promote intercellular communication, and enhance the survival rate of transplanted cells, in order to effectively improve the contraction function of bladder muscle and enhance the therapeutic efficacy of bladder weakness.

### SUMMARY OF THE INVENTION

Therefore, the present invention provides a cell preparation for use in the treatment of detrusor underactivity and method for preparing the same to solve the problems of the prior art.

The cell preparation for use in the treatment of detrusor underactivity, wherein the cell preparation comprises a cell aggregate, and the cell aggregate is formed by seeding a plurality of mesenchymal stem cells into a culture device comprising a plurality of culture wells, and culturing the mesenchymal stem cells on a surface of the culture wells, wherein each of the culture wells comprises a low-protein-adhesion coating.

Wherein, the mesenchymal stem cells are seeded at a density of 5.0 × 10⁴ cells/cm² to 1.0 × 10⁷ cells/cm² and cultured for 24 to 72 hours.

Wherein, each of the culture wells has a diameter of 200 µm to 500 µm and a depth of 100 µm to 200 µm.

Wherein, the cell aggregate comprises a plurality of target cells, and each cell aggregate comprises 30 to 600 of the target cells.

Wherein, after administration of the cell preparation, a post-treatment bladder tissue sample is compared with a pre-treatment bladder tissue sample, and a statistically significant change is observed in the expression level of at least one gene selected from the group consisting of: *Inhba, Kif5c, ANGPTL7, Mcpt1, Mcpt2, Pla2g2a, Prss35, Rasl2-9, Spp1, Sult1c2a,* and *Wif1.*

The present invention provides a method for preparing a cell preparation for use in the treatment of detrusor underactivity, comprising the following steps: seeding a plurality of mesenchymal stem cells into a culture device comprising a plurality of culture wells, wherein a surface of each of the culture wells comprises a low-protein-adhesion coating; and culturing the mesenchymal stem cells to form a cell aggregate.

Wherein, the mesenchymal stem cells are seeded at a density of 5.0 × 10⁴ cells/cm² to 1.0 × 10⁷ cells/cm² and cultured for 24 to 72 hours.

Wherein, each of the culture wells has a diameter of 200 µm to 500 µm and a depth of 100 µm to 200 µm.

Wherein, the cell aggregate comprises a plurality of target cells, and each cell aggregate comprises 30 to 600 of the target cells.

Wherein, the method for preparing a cell preparation for use in the treatment of detrusor underactivity, further the following step: isolating the formed cell aggregate from the culture device for collection.

In summary, the present invention provides a cell composition for use in the treatment of detrusor underactivity and method for preparing the same. The cell preparation of the present invention enables the formation of three-dimensional cell aggregates that promote intercellular interactions and physiological functionality, thereby enhancing post-transplantation cell viability and functional performance. Notably, the cell preparation effectively facilitates the repair and restoration of contractile function in bladder muscle tissue, improving the clinical symptoms associated with detrusor underactivity. Through specific cell culture conditions and a defined method of forming cell aggregates, the present invention overcomes the limitations of conventional two-dimensional culture methods, such as failure to simulate the in vivo microenvironment, restricted cell function, and low transplantation efficiency. Furthermore, the invention has been shown to significantly regulate the expression of multiple genes in bladder tissue, thus providing a safe and highly effective innovative cell therapy strategy for the treatment of detrusor underactivity.

### BRIEF DESCRIPTION OF THE APPENDED DRAWINGS

FIG. 1 is a graph showing the gene expression levels analyzed by quantitative polymerase chain reaction (qPCR) for cell aggregates cultured for 24 to 48 hours.
FIG. 2 is a comparative image showing bladder size after treatment with the cell aggregates in a detrusor underactivity animal model.
FIG. 3 is a graph illustrating changes in bladder pressure after treatment with the cell aggregates in a detrusor underactivity animal model.
FIG. 4 is a chart showing statistical results of multiple bladder function indicators after treatment with the cell aggregates in a detrusor underactivity animal model.
FIG. 5 is a flow diagram illustrating the steps of a method for preparing a cell preparation according to an embodiment of the present invention.
FIG. 6 is a flow diagram illustrating the steps of a method for preparing a cell preparation according to another embodiment of the present invention.

### DETAILED DESCRIPTION OF THE INVENTION

For the sake of the advantages, spirits and features of the present invention can be understood more easily and clearly, the detailed descriptions and discussions will be made later by way of the embodiments and with reference of the diagrams. It is worth noting that these embodiments are merely representative embodiments of the present invention, wherein the specific methods, devices, conditions, materials and the like are not limited to the embodiments of the present invention or corresponding embodiments. Moreover, the devices in the figures are only used to express their corresponding positions and are not drawing according to their actual proportion.

In the description of this specification, the description with reference to the terms "an embodiment", "another embodiment" or "part of an embodiment" means that a particular feature, structure, material or characteristic described in connection with the embodiment including in at least one embodiment of the present invention. In this specification, the schematic representations of the above terms do not necessarily refer to the same embodiment. Furthermore, the particular features, structures, materials or characteristics described may be combined in any suitable manner in one or more embodiments. Furthermore, the indefinite articles "a" and "an" preceding a device or element of the present invention are not limiting on the quantitative requirement (the number of occurrences) of the device or element. Thus, "a" should be read to include one or at least one, and a device or element in the singular also comprises the plural unless the number clearly refers to the singular.

According to the present invention, the term "mesenchymal stem cell (MSC)" refers to a heterogeneous population of stem cells derived from various sources and possessing multipotent differentiation potential. These cells can be induced to differentiate into various cell types, including myocytes, osteoblasts, chondrocytes, and adipocytes. The mesenchymal stem cells applied in the present invention may be obtained from multiple sources such as bone marrow, adipose tissue, umbilical cord, or other mesenchymal tissues. These cells exhibit favorable proliferative capacity and differentiation plasticity under in vitro conditions, making them suitable as foundational cell therapy materials in the field of regenerative medicine.

As used in this specification, unless explicitly stated otherwise, the term "treating" shall be interpreted broadly to include, but not be limited to, alleviating, improving, inhibiting, preventing, or reversing the symptoms or progression of detrusor underactivity. Specifically, treatment may involve enhancing the contractile function of the bladder smooth muscle to restore normal voiding activity and reduce the risk of complications associated with voiding dysfunction, thereby improving the patient's quality of life.

According to the present invention, the disclosed cell preparation may be formulated into a dosage form suitable for non-enteral administration. The dosage form may be selected from injectable formulations, including sterile aqueous solutions, suspensions, or lyophilized powders, and may be adjusted in concentration and dose according to clinical requirements. The cell preparation may be administered via intraperitoneal injection, subcutaneous injection, intramuscular injection, intravenous injection, or local intralesional injection. In particular, local injection into the bladder wall allows direct delivery of the cell aggregate to the target tissue, thereby improving tissue repair and functional restoration efficiency.

Furthermore, the cell preparation disclosed herein may optionally include one or more pharmaceutically acceptable carriers or excipients to maintain cellular viability and stability. The carriers or excipients may include water, saline, phosphate-buffered saline (PBS), sugar-containing solutions, or aqueous alcoholic solutions, and may further comprise stabilizers, antioxidants, gelling agents, suspending agents, or sustained-release agents to enhance storage stability and physicochemical properties during administration. The selection and optimization of such formulation components may be carried out based on the technical knowledge and experience of a person skilled in the art.

The present invention provides a use of a cell composition for use in the treatment of detrusor underactivity and method for preparing the same, wherein the cell preparation comprises a cell aggregate. The cell aggregate is formed by seeding a plurality of mesenchymal stem cells into a culture device comprising a plurality of culture wells, and culturing the mesenchymal stem cells on a surface of the culture wells, wherein each of the culture wells comprises a low-protein-adhesion coating. In the present embodiment, the number of mesenchymal stem cells seeded is in the range of 5.0 × 10⁴ cells/cm² to 1.0 × 10⁷ cells/cm², and the culture duration is 24 to 72 hours to promote natural cell aggregation and formation of a structured cell aggregate. The resulting cell aggregate can more accurately mimic the in vivo three-dimensional microenvironment, thereby enhancing cellular functionality and subsequent therapeutic outcomes. However, in practice, the cell density and culture duration are not limited to the aforementioned ranges and may be appropriately adjusted based on factors such as the type of mesenchymal stem cell used, its tissue source, growth characteristics, or specific therapeutic requirements in order to optimize aggregate formation and functionality.

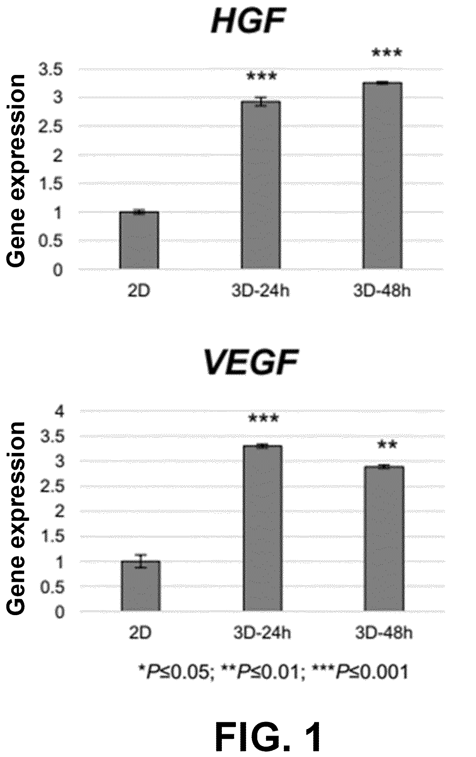

To address the impaired contractility of bladder smooth muscle in patients with detrusor underactivity, the inventors conducted a series of studies applying the cell aggregate to the treatment of detrusor underactivity, including the establishment of a mouse animal model for functional evaluation. In the studies, the cultured cell aggregates were injected into the bladder wall to assess their effects on bladder tissue repair and restoration of contractile function. Please refer to FIG. 1 to FIG. 4 for relevant experimental data and analysis results.

FIG. 1 is a graph showing the gene expression levels analyzed by quantitative polymerase chain reaction (qPCR) for cell aggregates cultured for 24 to 48 hours. The analyzed genes include hepatocyte growth factor (HGF), and vascular endothelial growth factor (VEGF). The inventors of the present invention employed qPCR to quantify the expression of the aforementioned genes in the cell aggregates. As shown in FIG. 1, the expression levels of HGF and VEGF were significantly elevated in the three-dimensional (3D) cell aggregates (3D-24h and 3D-48h groups) as compared to conventionally cultured two-dimensional (2D) cells. Since HGF and VEGF are key growth factors involved in tissue repair and angiogenesis, their upregulated expression indicates that the cell aggregates possess enhanced tissue regenerative potential. Please note: The statistical symbols shown in the figure indicate different levels of significance. *P* ≤ 0.05 (*) denotes statistical significance; *P* ≤ 0.01 (**) indicates high statistical significance; and *P* ≤ 0.001 (*) represents extremely high statistical significance.

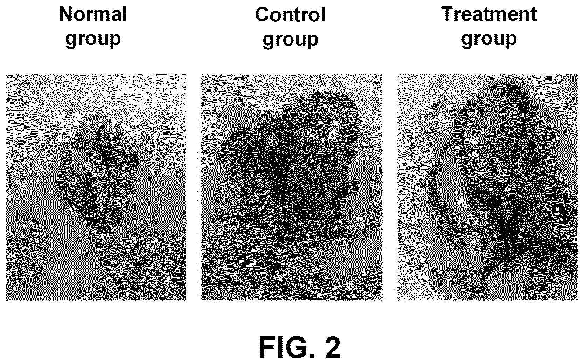

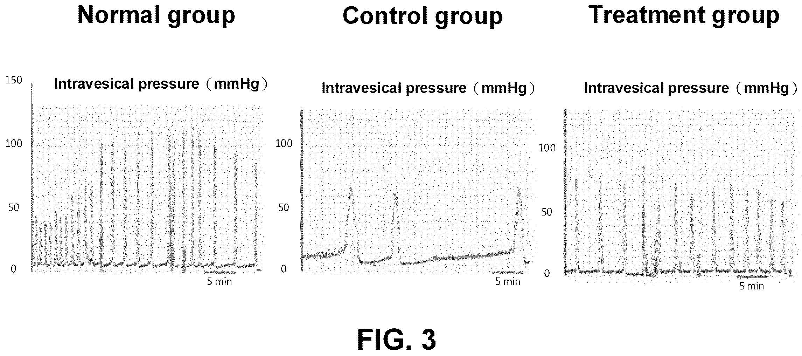

FIG. 2 is a comparative image showing bladder size after treatment with the cell aggregates in a detrusor underactivity animal model. FIG. 3 is a graph illustrating changes in bladder pressure after treatment with the cell aggregates in the detrusor underactivity animal model. In this study, the normal group refers to untreated animals, while both the control group and the treatment group were induced to exhibit detrusor underactivity symptoms using pharmacological methods. The treatment group subsequently received cell aggregate injections. As shown in FIG. 2, the bladder size of the treatment group was noticeably smaller than that of the untreated control group, suggesting that the cell aggregate treatment effectively reduced pathological bladder enlargement caused by impaired bladder contractility and promoted recovery of bladder muscle tissue structure. Referring to the measurement results in FIG. 3, the peak intravesical pressure during voiding in the treatment group was significantly higher than that in the control group, confirming that cell aggregate administration not only facilitated structural restoration but also improved bladder contractile function, thereby offering tangible technical support for treating detrusor underactivity.

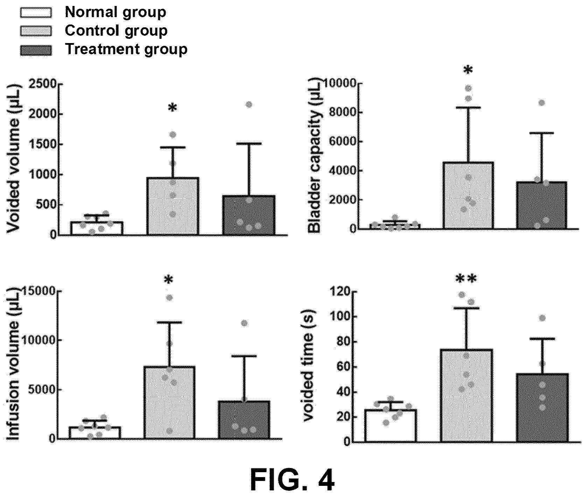

FIG.4 is a chart showing statistical results of multiple bladder function indicators after treatment with the cell aggregates in the detrusor underactivity animal model. As shown in FIG. 4, the multiple bladder function indicators include voided volume, bladder capacity, infusion volume, and voided time. In the detrusor underactivity control group, the voided volume, bladder capacity and infusion volume were significantly higher than those in the normal group, indicating that the bladder contractility and voiding efficiency were reduced, resulting in over-distension and difficulty in urination; in addition, the voided time was significantly longer, which is a typical sign of bladder weakness. In contrast, the treatment group exhibited a trend toward functional recovery as evidenced by significantly reduced voided volume, bladder capacity, and infusion volume relative to the untreated control group, indicating improved bladder contractility and restoration of both urine storage and voiding functions. Furthermore, voided time was significantly shortened, approaching the level of the normal group, suggesting enhanced voiding efficiency. Collectively, these results indicate that treatment with the cell aggregate effectively alleviates pathological bladder overdistension and delayed urination, and promotes reconstruction of bladder smooth muscle contractility, demonstrating clear clinical potential for the treatment of detrusor underactivity.

Based on the aforementioned experimental results, it can be confirmed that the cell aggregate of the present invention exhibits significant efficacy in promoting the repair of bladder muscle tissue and the restoration of contractile function. These findings demonstrate the therapeutic potential of the cell aggregate in the treatment of detrusor underactivity and provide feasibility support for its clinical application as a novel cell therapy strategy.

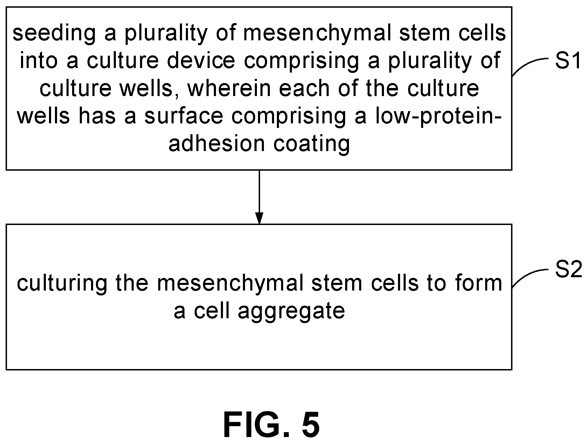

The present invention further provides a method for preparing a cell preparation for use in the treatment of detrusor underactivity, as illustrated in FIG. 5. FIG. 5 is a flow diagram illustrating the steps of a method for preparing a cell preparation for use in the treatment of detrusor underactivity according to an embodiment of the present invention. As shown in FIG. 5, the method comprises the following steps: Step S1: seeding a plurality of mesenchymal stem cells into a culture device comprising a plurality of culture wells, wherein each of the culture wells has a surface comprising a low-protein-adhesion coating; and Step S2: culturing the mesenchymal stem cells to form a cell aggregate.

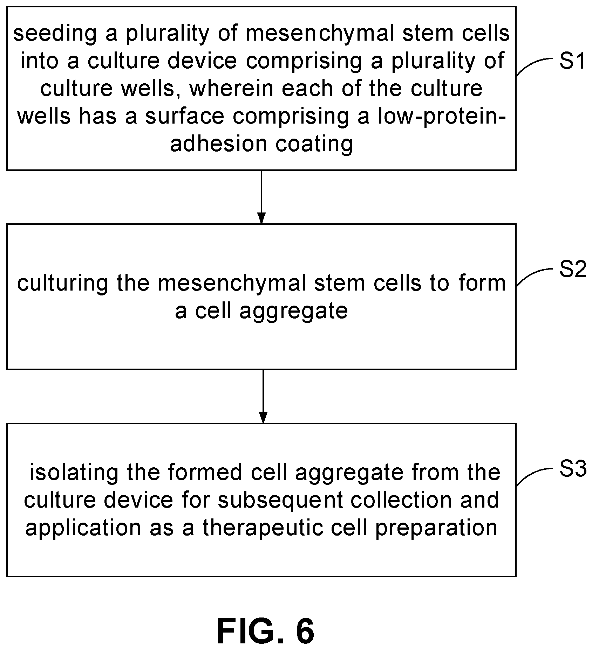

In another embodiment, the method can further comprise a separation step, as illustrated in FIG. 6. FIG. 6 is a flow diagram illustrating the steps of a method for preparing a cell preparation for use in the treatment of detrusor underactivity according to another embodiment of the present invention. As shown in FIG. 6, the method further comprises: Step S3: isolating the formed cell aggregate from the culture device for subsequent collection and application as a therapeutic cell preparation.

In this embodiment, the diameter of each culture well of the culture device is between 200 µm and 500 µm, and the depth is between 100 µm and 200 µm. By controlling the size of the culture wells, stable and uniform cell aggregates can be efficiently formed within localized microenvironments, thereby improving the homogeneity of cell culture and enhancing adaptability for subsequent transplantation. However, in practice, the diameter and depth of the culture wells can be adjusted depending on the cell type, cell size, or the desired aggregate dimensions, and are not limited to the specific ranges described above.

In this embodiment, each formed cell aggregate comprises a plurality of target cells, with the number of target cells per aggregate ranging from 30 to 600. By controlling the number of cells within each aggregate, structural stability and nutrient permeability can be balanced to avoid central necrosis and enhance post-transplantation cell viability and functional performance. Nonetheless, in practice, the number of cells per aggregate can be adjusted according to the cell type, culture conditions, or intended therapeutic use to achieve optimal biological activity and clinical efficacy.

In this embodiment, the cell preparation of the present invention was applied to a mouse model of detrusor underactivity. The bladder tissue samples were collected before and after treatment to assess gene expression changes. This gene-level evaluation further validated the therapeutic effects of the cell preparation on tissue repair and functional recovery. The results demonstrated that expression levels of at least one gene selected from the following group showed statistically significant changes: *Inhba* (Inhibin Beta A Chain), *Kif5c* (Kinesin Family Member 5C), *ANGPTL7* (Angiopoietin-related Protein 7-like), *Mcpt1* (Mast Cell Protease 1), *Mcpt2* (Mast Cell Protease 2), *Pla2g2a* (Phospholipase A2 Group IIA), *Prss35* (Protease, Serine 35), *Rasl2-9* (RAS-like, family 2, locus 9), *Spp1* (Secreted Phosphoprotein 1), *Sult1c2a* (Sulfotransferase Family 1C Member 2A), and *Wif1* (Wnt Inhibitory Factor 1). These findings confirm that the cell preparation of the present invention can effectively promote bladder tissue repair and functional recovery.

Additionally, to compare the gene expression profiles between the cell preparation of the present invention and mesenchymal stem cells (MSCs) cultured as single cells under conventional two-dimensional (2D) conditions, a gene expression analysis was conducted. The results demonstrated that the cell preparation of the present invention exhibited significantly elevated expression levels of key genes, including VEGF (Vascular Endothelial Growth Factor), FGF 10 (Fibroblast Growth Factor 10), FGF 14 (Fibroblast Growth Factor 14), PDGFD (Platelet-Derived Growth Factor D), BMP2 (Bone Morphogenetic Protein 2), CSF3 (Colony Stimulating Factor 3), CXCR4 (C-X-C Chemokine Receptor Type 4), STC1 (Stanniocalcin 1), MMP2 (Matrix Metalloproteinase-2), ELN (Elastin), and IGF1 (Insulin-Like Growth Factor 1), *MMP10* (Matrix Metalloproteinase-*10), AREG* (Amphiregulin), and *IL-1b* (Interleukin-1 beta), as compared to conventional single-cell MSCs. The 3D aggregate structure formed using the method of the present invention effectively promotes the upregulation of these critical genes, thereby enhancing the angiogenic, tissue-regenerative, and anti-fibrotic properties of the cells, as well as improving intercellular signaling and overall physiological activity. Moreover, the cell preparation of the present invention demonstrated a significantly prolonged in vivo retention time compared to conventional 2D-cultured MSCs, indicating improved post-transplantation survival and tissue engraftment capacity. This enhanced retention further supports the feasibility of the present invention as a practical and clinically applicable cell-based therapeutic strategy. In conclusion, the cell preparation of the present invention exhibits superior performance over conventional 2D-cultured MSCs in terms of in vivo microenvironment simulation, functional gene expression, retention characteristics, and clinical potential.

In summary, the present invention provides a cell composition for use in the treatment of detrusor underactivity and method for preparing the same. The cell preparation of the present invention enables the formation of three-dimensional cell aggregates that promote intercellular interactions and physiological functionality, thereby enhancing post-transplantation cell viability and functional performance. Notably, the cell preparation effectively facilitates the repair and restoration of contractile function in bladder muscle tissue, improving the clinical symptoms associated with detrusor underactivity. Through specific cell culture conditions and a defined method of forming cell aggregates, the present invention overcomes the limitations of conventional two-dimensional culture methods, such as failure to simulate the in vivo microenvironment, restricted cell function, and low transplantation efficiency. Furthermore, the invention has been shown to significantly regulate the expression of multiple genes in bladder tissue, thus providing a safe and highly effective innovative cell therapy strategy for the treatment of detrusor underactivity.

With the examples and explanations mentioned above, the features and spirits of the invention are hopefully well described. More importantly, the present invention is not limited to the embodiment described herein. Those skilled in the art will readily observe that numerous modifications and alterations of the device may be made while retaining the teachings of the invention. Accordingly, the above disclosure should be construed as limited only by the metes and bounds of the appended claims.

## Claims

1. A cell preparation for use in the treatment of detrusor underactivity, wherein the cell preparation comprises a cell aggregate, and the cell aggregate is formed by seeding a plurality of mesenchymal stem cells into a culture device comprising a plurality of culture wells, and culturing the mesenchymal stem cells on a surface of the culture wells, wherein each of the culture wells comprises a low-protein-adhesion coating.

2. The cell preparation for use in the treatment of detrusor underactivity according to claim 1, wherein the mesenchymal stem cells are seeded at a density of 5.0 × 10⁴ cells/cm² to 1.0 × 10⁷ cells/cm² and cultured for 24 to 72 hours.

3. The cell preparation for use in the treatment of detrusor underactivity according to claim 1, wherein each of the culture wells has a diameter of 200 µm to 500 µm and a depth of 100 µm to 200 µm.

4. The cell preparation for use in the treatment of detrusor underactivity according to claim 1, wherein the cell aggregate comprises a plurality of target cells, and each cell aggregate comprises 30 to 600 of the target cells.

5. The cell preparation for use in the treatment of detrusor underactivity according to claim 1, wherein after administration of the cell preparation, a post-treatment bladder tissue sample is compared with a pre-treatment bladder tissue sample, and a statistically significant change is observed in the expression level of at least one gene selected from the group consisting of: *Inhba, Kif5c, ANGPTL7, Mcpt1, Mcpt2, Pla2g2a, Prss35, Rasl2-9, Spp1, Sult1c2a,* and *Wif1.*

6. A method for preparing a cell preparation for use in the treatment of detrusor underactivity, comprising the following steps:
seeding a plurality of mesenchymal stem cells into a culture device comprising a plurality of culture wells, wherein a surface of each of the culture wells comprises a low-protein-adhesion coating; and
culturing the mesenchymal stem cells to form a cell aggregate.

7. The method according to claim 6, wherein the mesenchymal stem cells are seeded at a density of 5.0 × 10⁴ cells/cm² to 1.0 × 10⁷ cells/cm² and cultured for 24 to 72 hours.

8. The method according to claim 6, wherein each of the culture wells has a diameter of 200 µm to 500 µm and a depth of 100 µm to 200 µm.

9. The method according to claim 6, wherein the cell aggregate comprises a plurality of target cells, and each cell aggregate comprises 30 to 600 of the target cells.

10. The method according to claim 6, further the following step:
isolating the formed cell aggregate from the culture device for collection.
